# EUROPEAN PATENT APPLICATION

(11) **EP 4 230 192 A1**
(43) Date of publication of application: **23.08.2023**
(21) Application number: 21906544.8
(22) Date of filing: 13.12.2021
(51) Int. Cl.: A61K 8/49, A61Q 19/00, A61Q 19/08, C12N 15/09

(54) **EPIDERMAL STEM CELL PROLIFERATION-PROMOTING AGENT**

(30) Priority: 15.12.2020 JP 2020207552
(71) Applicant: Shiseido Company, Ltd., Chuo-ku Tokyo 104-0061 (JP)
(72) Inventor: TOBITA Ryozo, Tokyo 104-0061 (JP); IRIYAMA Shunsuke, Tokyo 104-0061 (JP); YAMADA Minami, Tokyo 104-0061 (JP)
(74) Representative: Ter Meer Steinmeister & Partner
(86) International application number: PCT/JP2021/045725
(87) International publication number: WO 2022/131182

(57) **Abstract**

The disclosure of the present invention relates to the provision of compositions effective for improving skin conditions from the viewpoint of maintaining or improving quality of life (QOL) in terms of both health and beauty. More specifically, the disclosure of the present invention relates to compositions comprising 1-(2-hydroxyethyl)-2-imidazolidinone or a derivative thereof and pyridinecarboxamide. As they promote the increase of epidermal stem cells and facilitate the proliferation of keratinocytes, they can be suitably used as a cosmetic composition such as cosmetics that are effective in improving skin conditions.

## Description

### Technical Field

The present invention relates to a composition for external use on the skin, and more particularly to a topical skin composition comprising 1-(2-hydroxyethyl)-2-imidazolidinone or a derivative thereof and pyridinecarboxamide.

### Background Art

With the arrival of a full-fledged aging society, research and development on aging mechanisms are becoming active. Among them, the skin forms the outermost layer of the human body, plays an important role in bioprotection, and is a particularly important organ in terms of beauty because its appearance can be visually perceived. From the viewpoint of maintaining or improving quality of life (QOL) in terms of both health and beauty, there has been an increase of interests in skin aging prevention in recent years.

For the purpose of skin aging prevention, topical skin preparations such as cosmetics have long been blended with moisturizing agents that are expected to improve the skin's ability to retain moisture, vitamins E and C which are expected to act as antioxidants, and the like. Further, as a result of recent studies, it has become clear that the reduction and degeneration of matrix components in the skin are greatly involved in wrinkles, sagging, and loss of firmness associated with aging.

In particular, the decrease in collagen, which is the main component of the matrix, is related to the sagging and firmness of the skin, and the degradation and degeneration of elastin caused by the expression of the elastin degrading enzyme is thought to be the cause of the decrease in elasticity. The collagen production promoting agents, collagen degrading enzyme-inhibiting agents, elastin degrading enzyme-inhibiting agents, and the like are blended as anti-aging agents. Nevertheless, although these drugs have certain effects, they have not yet shown sufficient effects.

Patent Document 1 discloses an anti-aging skin preparation for external use that is effective in restoring and maintaining skin firmness and elasticity, reducing wrinkles and sagging, and reducing pigmentation, and is capable of restoring and maintaining a youthful skin condition, wherein the anti-aging skin external preparation is characterized by comprising niacin and ubiquinone.

Patent Document 2 discloses a cyclic carboxamide derivative having a specific structure, as a drug effective for preventing and suppressing skin aging from the viewpoint of the connection between heparanase and skin aging, and as a component of a whitening agent effective in preventing and suppressing pigmentation such as spots, freckles, and dullness.

### Citation List

### Patent Documents

Patent Document 1: JP 2005-298370
Patent Document 2: JP 2014-111640

Non-patent Document 1: Vlodavsky I., et. al., Semin Cancer Biol., 2002;12(2):121-129

### Summary of the Invention

### Problems to be Solved by the Invention

An objective of the present invention is to find a composition that is effective in improving skin conditions from the viewpoint of maintaining or improving quality of life (QOL) in terms of both health and beauty.

### Means for Solving the Problems

As a result of intensive studies, the inventors of the present invention have found that a composition comprising 1-(2-hydroxyethyl)-2-imidazolidinone or a derivative thereof and pyridinecarboxamide exhibits an effect of promoting epidermal stem cell proliferation.

Accordingly, the disclosure of the present invention provides the embodiments below.
(1) A topical skin composition comprising 1-(2-hydroxyethyl)-2-imidazolidinone or a derivative thereof and pyridinecarboxamide as active ingredients.
(2) The composition according to (1), which is a cosmetic composition.
(3) The composition according to (1) or (2), which is for use in promoting the proliferation of epidermal stem cells.
(4) The composition according to (3), wherein the epidermal stem cells are MCSP-positive cells.
(5) The composition according to any one of (1) to (4), which promotes keratinocyte proliferation.
(6) The composition according to any one of (1) to (5), wherein the 1-(2-hydroxyethyl)-2-imidazolidinone or a derivative thereof is a cyclic carboxamide derivative represented by the general formula (I) below: 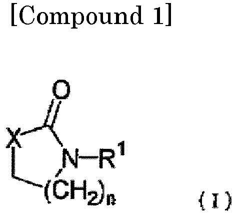 wherein, n is an integer of 1 to 3, R¹ is a hydrogen atom or a hydrocarbon group having 1 to 6 carbon atoms which may be substituted with a hydroxyl group, and X is -CH₂- or a group represented by -N(R²)-, and R² refers to a hydrogen atom or a hydrocarbon group having 1 to 6 carbon atoms which may be substituted with a hydroxyl group.
(7) The composition according to any one of (1) to (6), wherein the pyridinecarboxamide is selected from the group consisting of 2-pyridinecarboxamide, 3-pyridinecarboxamide, 4-pyridinecarboxamide, and a mixture thereof.
(8) A cosmetic method, comprising applying to the skin a composition containing 1-(2-hydroxyethyl)-2-imidazolidinone or a derivative thereof and pyridinecarboxamide.
(9) The composition according to any one of (1) to (7), which is for use in promoting the expression of collagen in the papillary dermis.
(10) The composition according to any one of (1) to (7), which is for use in promoting the expression of collagen in the dermis.

### Effect of the Invention

Since the composition according to the disclosure of the present invention promotes the increase of epidermal stem cells and facilitates the proliferation of keratinocytes, it can be suitably used, for example, as a cosmetic composition such as cosmetics effective in improving skin conditions.

### Brief Description of the Drawings

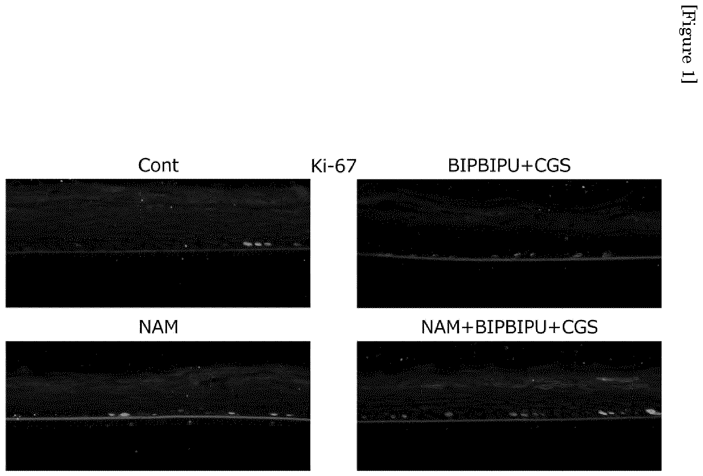

[Fig. 1] Figure 1 is an image showing the results of investigating the effects of a combination of nicotinamide (NAM), MMP-9 inhibitor CGS27023A and heparanase inhibitor BIPBIPU on keratinocyte proliferation based on the expression of Ki-67 in a three dimensional epidermal skin model. Cont is the untreated control; NAM is the nicotinamide treatment; BIPBIPU + CGS is the treatment with a combination of BIPBIPU and CGS, and NAM + BIPBIPU + CGS is the treatment with a combination of NAM, BIPBIPU and CGS.

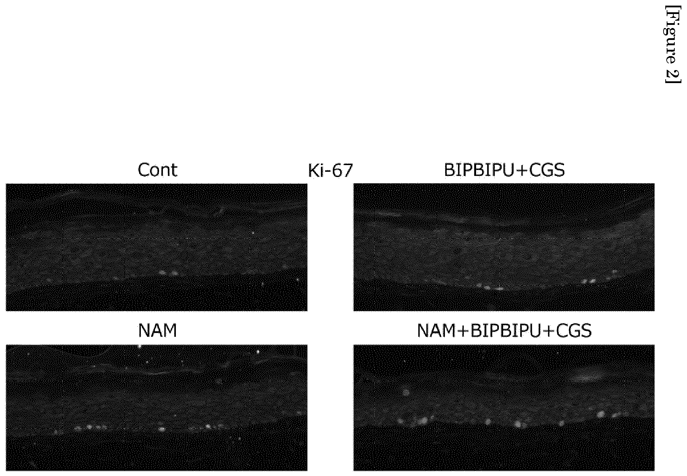

[Fig. 2] Figure 2 is an image showing the results of investigating the effects of a combination of nicotinamide (NAM), MMP-9 inhibitor CGS27023A and heparanase inhibitor BIPBIPU on keratinocyte proliferation based on the expression of Ki-67 in a dermis-containing three dimensional skin model. Cont is the untreated control; NAM is the nicotinamide treatment; BIPBIPU + CGS is the treatment with a combination of BIPBIPU and CGS, and NAM + BIPBIPU + CGS is the treatment with a combination of NAM, BIPBIPU and CGS.

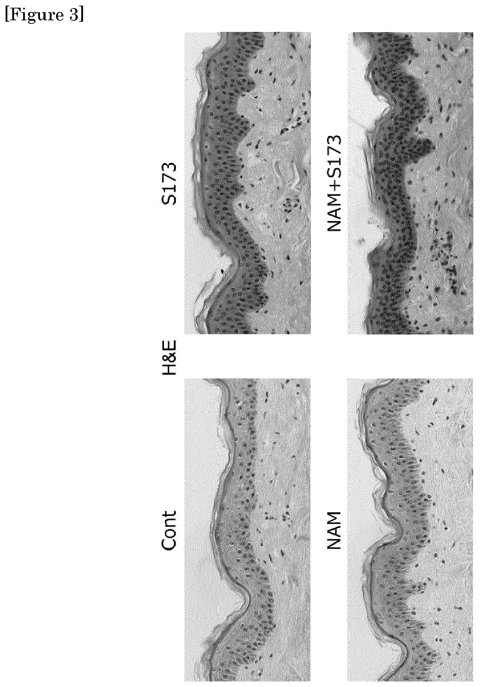

[Fig. 3] Figure 3 is an image showing the results of investigating the effects of nicotinamide (NAM) and 1-(2-hydroxyethyl)-2-imidazolidinone (S173) on fresh skin samples by H&E staining. Cont is the untreated control; NAM is the nicotinamide treatment alone; S173 is the S173 treatment alone; and NAM + S173 is the treatment with a combination of NAM and S173.

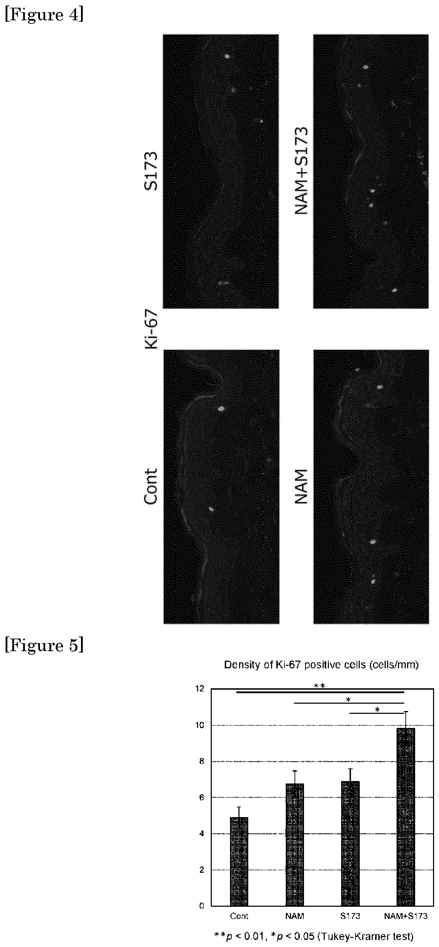

[Fig. 4] Figure 4 is an image showing the results of investigating the effects of nicotinamide (NAM) and 1-(2-hydroxyethyl)-2-imidazolidinone (S173) on fresh skin samples based on the expression of Ki-67, which is a marker of the cell proliferation. Cont is the untreated control; NAM is the nicotinamide treatment alone; S173 is the S173 treatment alone; and NAM + S173 is the treatment with a combination of NAM and S173.
[Fig. 5] Figure 5 is a graph showing the results of quantifying the number of Ki-67-positive cells per basement membrane length based on the immunostaining results. Cont is the untreated control; NAM is the nicotinamide treatment alone; S 173 is the S173 treatment alone; and NAM + S173 is the treatment with a combination of NAM and S173.

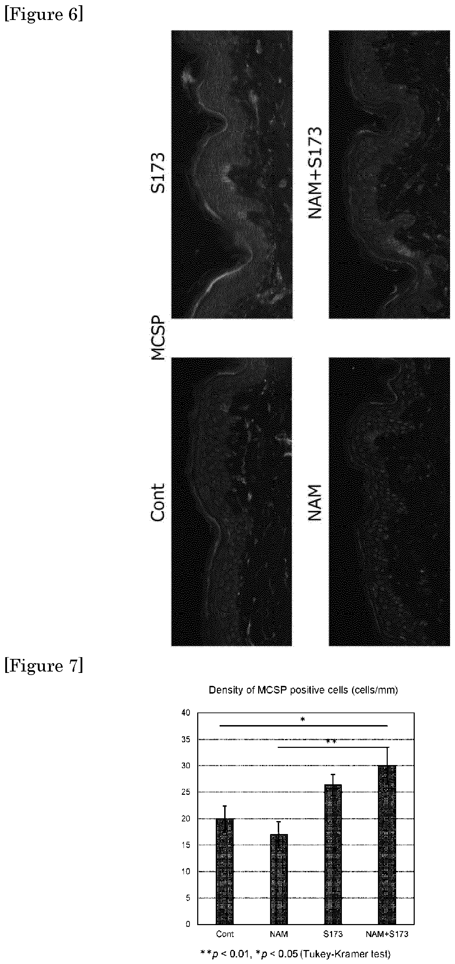

[Fig. 6] Figure 6 is an image showing the results of investigating the effects of nicotinamide (NAM) and 1-(2-hydroxyethyl)-2-imidazolidinone (S173) on epidermal stem cells based on the expression of MCSP. Cont is the untreated control; NAM is the nicotinamide treatment alone; S 173 is the S173 treatment alone; and NAM + S173 is the treatment with a combination of NAM and S173.
[Fig. 7] Figure 7 is a graph showing the results of quantifying the number of MCSP-positive cells per basement membrane length based on the immunostaining results. Cont is the untreated control; NAM is the nicotinamide treatment alone; S 173 is the S173 treatment alone; and NAM + S173 is the treatment with a combination of NAM and S173.

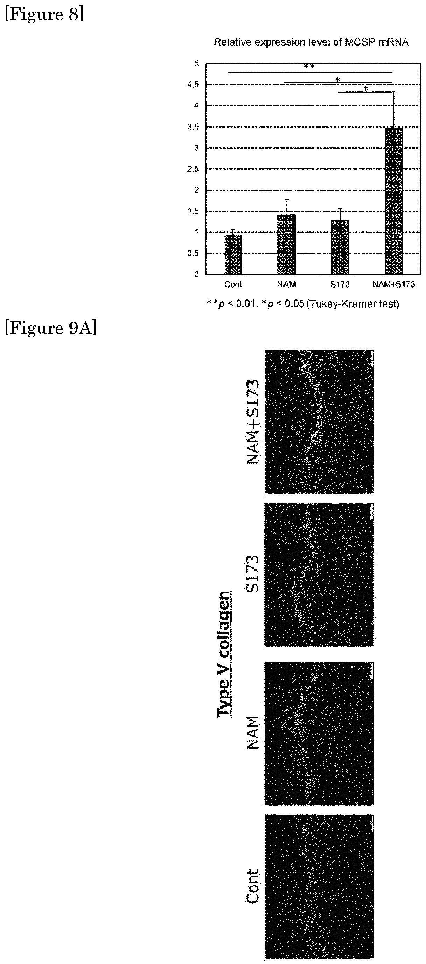

[Fig. 8] Figure 8 is a graph showing the results of examining the relative expression levels of MCSP mRNA by qPCR analysis. Cont is the untreated control; NAM is the nicotinamide treatment alone; S 173 is the S173 treatment alone; and NAM + S173 is the treatment with a combination of NAM and S173.
[Fig. 9A] Figure 9A is an image showing the results of investigating the effects of nicotinamide (NAM) and 1-(2-hydroxyethyl)-2-imidazolidinone (S173) on the production of Type V collagen (Type V collagen). Cont is the untreated control; NAM is the nicotinamide treatment alone; S 173 is the S173 treatment alone; and NAM + S173 is the treatment with a combination of NAM and S173.

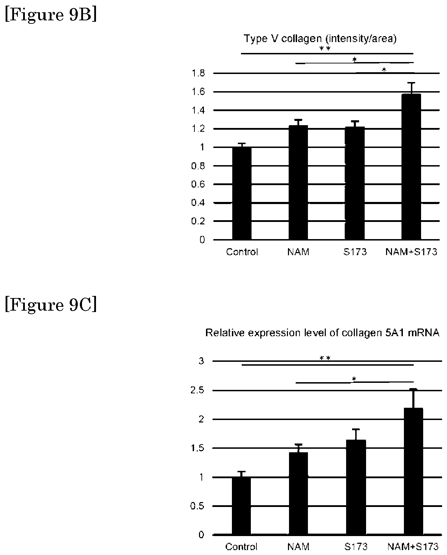

[Fig. 9B] Figure 9B is a graph quantifying the staining shown in Figure 9A (**p < 0.01, *p < 0.05; Tukey-Kramer test). Cont is the untreated control; NAM is the nicotinamide treatment alone; S173 is the S173 treatment alone; and NAM + S173 is the treatment with a combination of NAM and S173.
[Fig. 9C] Figure 9C is a graph showing the analysis results of collagen 5A1 gene expression (**p < 0.01, *p < 0.05 ; Tukey-Kramer test). Cont is the untreated control; NAM is the nicotinamide treatment alone; S173 is the S173 treatment alone; and NAM + S173 is the treatment with a combination of NAM and S173.

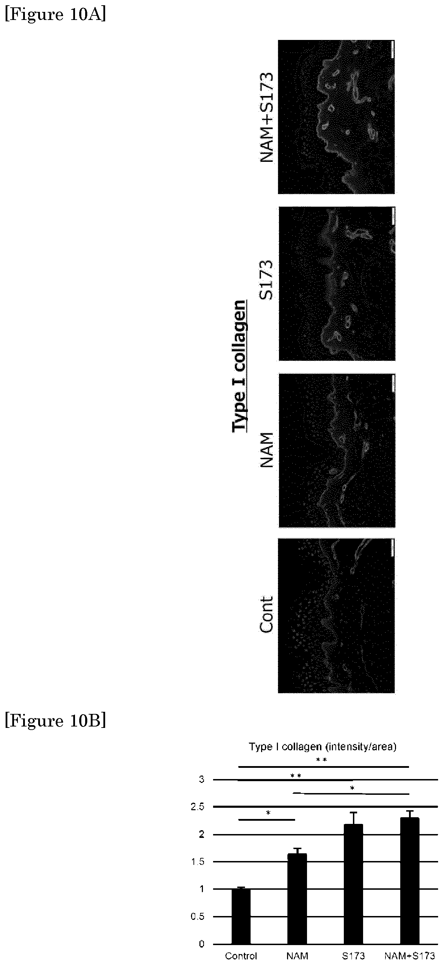

[Fig. 10A] Figure 10A is an image showing the results of investigating the effects of nicotinamide (NAM) and 1-(2-hydroxyethyl)-2-imidazolidinone (S173) on the production of Type I collagen (Type I collagen). Cont is the untreated control; NAM is the nicotinamide treatment alone; S173 is the S173 treatment alone; and NAM + S173 is the treatment with a combination of NAM and S173.
[Fig. 10B] Figure 10B is a graph quantifying the staining shown in Figure 10A (**p < 0.01, *p < 0.05; Tukey-Kramer test). Cont is the untreated control; NAM is the nicotinamide treatment alone; S173 is the S173 treatment alone; and NAM + S173 is the treatment with a combination of NAM and S173.

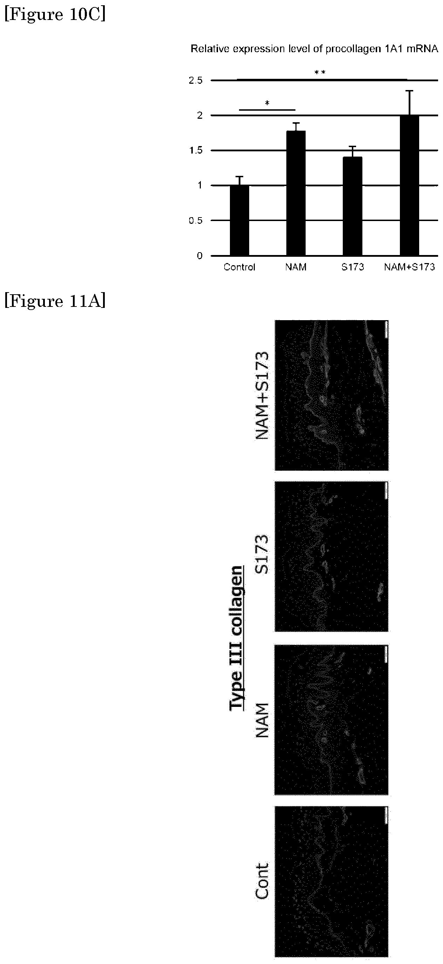

[Fig. 10C] Figure 10C is a graph showing the analysis results of procollagen 1A1 gene expression (**p < 0.01, *p < 0.05; Tukey-Kramer test). Cont is the untreated control; NAM is the nicotinamide treatment alone; S173 is the S173 treatment alone; and NAM + S173 is the treatment with a combination of NAM and S173.
[Fig. 11A] Figure 11A is an image showing the results of investigating the effects of nicotinamide (NAM) and 1-(2-hydroxyethyl)-2-imidazolidinone (S173) on the production of Type III collagen (Type III collagen). Cont is the untreated control; NAM is the nicotinamide treatment alone; S173 is the S173 treatment alone; and NAM + S173 is the treatment with a combination of NAM and S173.

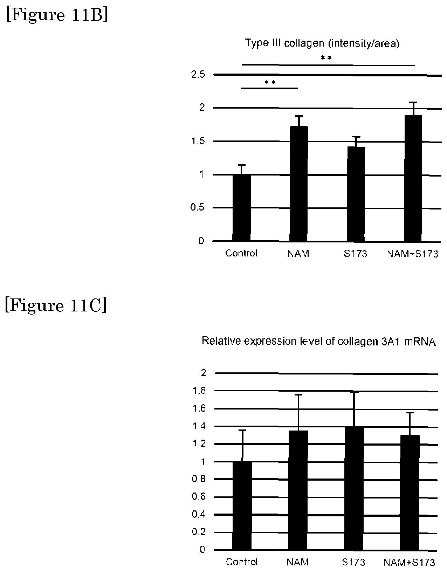

[Fig. 11B] Figure 11B is a graph quantifying the staining shown in Figure 11A (** p < 0.01, * p < 0.05; Tukey-Kramer test). Cont is the untreated control; NAM is the nicotinamide treatment alone; S173 is the S173 treatment alone; and NAM + S173 is the treatment with a combination of NAM and S173.
[Fig. 11C] Figure 11C is a graph showing the analysis results of collagen 3A1 gene expression (**p < 0.01, *p < 0.05; Tukey-Kramer test). Cont is the untreated control; NAM is the nicotinamide treatment alone; S173 is the S173 treatment alone; and NAM + S173 is the treatment with a combination of NAM and S173.

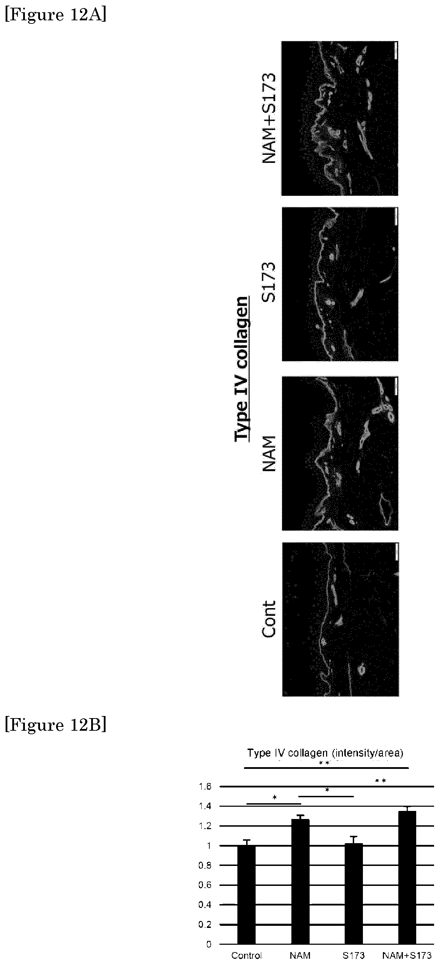

[Fig. 12A] Figure 12A is an image showing the results of investigating the effects of nicotinamide (NAM) and 1-(2-hydroxyethyl)-2-imidazolidinone (S173) on the production of Type IV collagen (Type IV collagen). Cont is the untreated control; NAM is the nicotinamide treatment alone; S173 is the S173 treatment alone; and NAM + S173 is the treatment with a combination of NAM and S173.
[Fig. 12B] Figure 12B is a graph quantifying the staining shown in Figure 12A (**p < 0.01, *p < 0.05; Tukey-Kramer test). Cont is the untreated control; NAM is the nicotinamide treatment alone; S173 is the S173 treatment alone; and NAM + S173 is the treatment with a combination of NAM and S173.

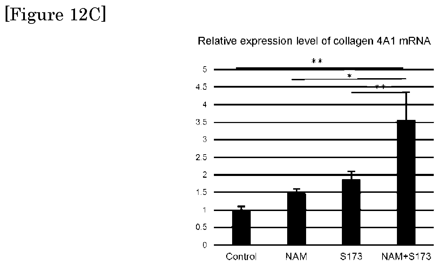

[Fig. 12C] Figure 12C is a graph showing the analysis results of collagen 4A1 gene expression (**p < 0.01, *p < 0.05; Tukey-Kramer test). Cont is the untreated control; NAM is the nicotinamide treatment alone; S173 is the S173 treatment alone; and NAM + S173 is the treatment with a combination of NAM and S173.

### Mode for Carrying Out the Invention

### Composition for external application to the skin

One embodiment in the disclosure of the present invention relates to a composition for external application to the skin which comprises 1-(2-hydroxyethyl)-2-imidazolidinone (HEI) or a derivative thereof and pyridinecarboxamide as active ingredients. Such compositions are preferably prepared as cosmetic compositions.

1-(2-hydroxyethyl)-2-imidazolidinone (HEI) is a compound with the structure below. In the present specification, 1-(2-hydroxyethyl)-2-imidazolidinone (HEI) is sometimes referred to as S173.

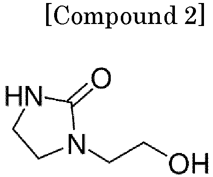

A derivative of 1-(2-hydroxyethyl)-2-imidazolidinone can be, for example, a compound represented by the general formula (I) below: 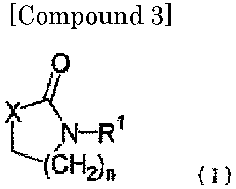 wherein, n is an integer of 1 to 3, R¹ is a hydrogen atom or a hydrocarbon group having 1 to 6 carbon atoms which may be substituted with a hydroxyl group, and X is -CH₂- or a group represented by -N(R²)-, and R² refers to a hydrogen atom or a hydrocarbon group having 1 to 6 carbon atoms which may be substituted with a hydroxyl group.

1-(2-hydroxyethyl)-2-imidazolidinone and derivatives thereof are known to act as inhibitors of the heparanase activity (see JP 2014-111640 (Patent Document 2)). Heparanase is an enzyme that exists in various cells and specifically degrades the heparan sulfate chains of various heparan sulfate proteoglycans. In the skin, epidermal keratinocytes, dermal fibroblasts, vascular endothelial cells, and the like, which constitute the epidermis, are produced. It is known that its production is increased in various cancer cells, and its association with cancer malignancy has been suggested. It is known that cancer cells with high heparanase production have a high metastatic potential and a high ability to induce angiogenesis (Vlodavsky I., et. al., Semin Cancer Biol., 2002;12(2):121-129 (see Non-patent Document 1)).

Heparan sulfate proteoglycans function to accumulate heparan sulfate-binding growth factors (bFGF, HGF, VEGF, HB-EGF, etc.) extracellularly. Perlecan, which is a type of heparan sulfate proteoglycan, is also present in the epidermal basement membrane at the boundary between the epidermis and dermis. In the skin, the transfer of growth factors between the epidermis and dermis is controlled by the binding of the heparan sulfate-binding growth factors to the epidermal basement membrane. Further, perlecan present in the epidermal basement membrane also regulates the action of growth factors on epidermal basal cells bound to the basement membrane, which has been shown to be essential for good epidermal proliferation and differentiation.

Further, 1-(2-hydroxyethyl)-2-imidazolidinone (HEI) is also known to act as an MMP (matrix metalloproteinase) inhibitor. HEI, for example, inhibits MMP-9 but does not affect the activities of MMP-1 and MMP-2.

Pyridinecarboxamides are compounds with the molecular formula C₆H₆N₂O and include the following compounds with different positions of the carboxamide group: 2-pyridinecarboxamide (picolinamide), 3-pyridinecarboxamide (nicotinamide), and 4-pyridinecarboxamide (isonicotinamide). In the embodiments pertaining to the disclosure of the present invention, any of 2-pyridinecarboxamide (picolinamide), 3-pyridinecarboxamide (nicotinamide), and 4-pyridinecarboxamide (isonicotinamide), or a mixture thereof, may be used. In some embodiments, 3-pyridinecarboxamide (nicotinamide) is suitably used. The structure of 3-pyridinecarboxamide (nicotinamide) is shown below.

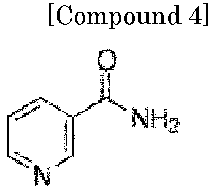

As detailed in the Examples section, the inventors of the present invention have now found that compositions comprising 1-(2-hydroxyethyl)-2-imidazolidinone or a derivative thereof and pyridinecarboxamide are effective in promoting an increase in epidermal stem cells and an increase in keratinocytes. The increase in these cells can be evaluated, for example, by immunohistochemical staining. For example, epidermal stem cells can be identified as MCSP-positive cells. Thus, one embodiment of the disclosure of the present invention relates to compositions characterized in that they comprise 1-(2-hydroxyethyl)-2-imidazolidinone or a derivative thereof and pyridinecarboxamide as active ingredients for use in promoting an increase in epidermal stem cells. Such compositions can preferably be prepared as topical skin compositions.

In other words, the composition according to the disclosure of the present invention can also be expressed as an epidermal stem cell proliferation promoting agent comprising 1-(2-hydroxyethyl)-2-imidazolidinone or a derivative thereof and pyridinecarboxamide as active ingredients. Compositions and agents according to the disclosure of the present invention may be used for non-therapeutic cosmetic applications, in particular. Also, from another aspect, an embodiment of the disclosure of the present invention relates to the use of a composition comprising 1-(2-hydroxyethyl)-2-imidazolidinone or a derivative thereof and pyridinecarboxamide for promoting epidermal stem cell proliferation. In such uses, 1-(2-hydroxyethyl)-2-imidazolidinone or its derivative and pyridinecarboxamide may be used in the form of a composition in combination with further components. From yet another aspect, one embodiment of the disclosure of the present invention relates to the use of 1-(2-hydroxyethyl)-2-imidazolidinone or a derivative thereof and pyridinecarboxamide in the manufacture of an epidermal stem cell proliferation promoting agent. From yet another aspect, one embodiment of the disclosure of the present invention relates to a method for promoting the increase of epidermal stem cells, which comprises administering to a subject (for example, a human) a composition comprising 1-(2-hydroxyethyl)-2-imidazolidinone or a derivative thereof and pyridinecarboxamide.

In addition, one embodiment according to the disclosure of the present invention relates to a composition characterized by comprising 1-(2-hydroxyethyl)-2-imidazolidinone or a derivative thereof and pyridinecarboxamide as active ingredients for use in facilitating the proliferation of keratinocytes. Such compositions are preferably prepared as skin compositions for external use.

In other words, the composition according to the disclosure of the present invention can also be expressed as a keratinocyte proliferation promoting agent comprising 1-(2-hydroxyethyl)-2-imidazolidinone or a derivative thereof and pyridinecarboxamide as active ingredients. Compositions and agents according to the disclosure of the present invention may be used for non-therapeutic cosmetic applications, in particular. From another aspect, one embodiment of the disclosure of the present invention relates to the use of a composition comprising 1-(2-hydroxyethyl)-2-imidazolidinone or a derivative thereof and pyridinecarboxamide for promoting keratinocyte proliferation. In such uses, 1-(2-hydroxyethyl)-2-imidazolidinone or its derivatives and pyridinecarboxamide may be used in the form of a composition in combination with further components. From yet another aspect, one embodiment of the disclosure of the present invention relates to the use of 1-(2-hydroxyethyl)-2-imidazolidinone or a derivative thereof and pyridinecarboxamide in the manufacture of a keratinocyte proliferation promoting agent. From yet another aspect, one embodiment of the disclosure of the present invention relates to a method of promoting keratinocyte proliferation, comprising administering a composition comprising 1-(2-hydroxyethyl)-2-imidazolidinone or a derivative thereof and pyridinecarboxamide to a subject (for example, a human).

Furthermore, one embodiment according to the disclosure of the present invention relates to a composition characterized by comprising 1-(2-hydroxyethyl)-2-imidazolidinone or a derivative thereof and pyridinecarboxamide as active ingredients for use in the promotion of collagen expression in the papillary dermis. The collagen can be Type I, III, IV, or V collagen. Such compositions are preferably prepared as skin compositions for external use.

In other words, the composition according to the disclosure of the present invention can be expressed as an agent that promotes the expression of collagen in the papillary dermis, comprising 1-(2-hydroxyethyl)-2-imidazolidinone or a derivative thereof and pyridinecarboxamide as active ingredients. Compositions and agents according to the disclosure of the present invention may be used for non-therapeutic cosmetic applications, in particular. Further, from another aspect, one embodiment of the disclosure of the present invention relates to the use of a composition comprising 1-(2-hydroxyethyl)-2-imidazolidinone or a derivative thereof and pyridinecarboxamide for promoting collagen expression in the papillary dermis. In such uses, 1-(2-hydroxyethyl)-2-imidazolidinone or its derivatives and pyridinecarboxamide may be used in the form of a composition in combination with further components. From yet another aspect, one embodiment of the disclosure of the present invention relates to the use of 1-(2-hydroxyethyl)-2-imidazolidinone or a derivative thereof and pyridinecarboxamide in the manufacture of an agent for promoting collagen expression in the papillary dermis. From yet another aspect, one embodiment of the disclosure of the present invention relates to a method of promoting collagen expression in the papillary dermis, comprising administering a composition comprising 1-(2-hydroxyethyl)-2-imidazolidinone or a derivative thereof and pyridinecarboxamide to a subject (for example, a human).

Furthermore, one embodiment according to the disclosure of the present invention comprises 1-(2-hydroxyethyl)-2-imidazolidinone or a derivative thereof and pyridinecarboxamide as active ingredients for use in promoting collagen expression in the dermis. The collagen can be Type I, Type III, Type IV, or Type V collagen. Such compositions are preferably prepared as skin compositions for external use.

In other words, the composition according to the disclosure of the present invention comprises 1-(2-hydroxyethyl)-2-imidazolidinone or a derivative thereof and pyridinecarboxamide as active ingredients, and it can also be presented as an agent for promoting collagen expression in the dermis. Compositions and agents according to the disclosure of the present invention may be used for non-therapeutic cosmetic applications, in particular. From another aspect, an embodiment of the disclosure of the present invention relates to a composition comprising 1-(2-hydroxyethyl)-2-imidazolidinone or a derivative thereof and pyridinecarboxamide for promoting collagen expression in the dermis. In such uses, 1-(2-hydroxyethyl)-2-imidazolidinone or its derivatives and pyridinecarboxamide may be used in the form of a composition in combination with further components. From yet another aspect, one embodiment of the disclosure of the present invention relates to the use of 1-(2-hydroxyethyl)-2-imidazolidinone or a derivative thereof and pyridinecarboxamide in the manufacture of an agent for promoting collagen expression in the dermis. From yet another aspect, one embodiment of the disclosure of the present invention relates to a method for promoting the collagen expression in the dermis, comprising administering a composition comprising 1-(2-hydroxyethyl)-2-imidazolidinone or a derivative thereof and pyridinecarboxamide to a subject (for example, a human).

### Cosmetic method

One embodiment of the disclosure of the present invention relates to a cosmetic method characterized in applying to the skin a composition comprising 1-(2-hydroxyethyl)-2-imidazolidinone or a derivative thereof and nicotinamide. The subject can be any animal, and is preferably a human. The cosmetic method according to the disclosure of the present invention is a non-therapeutic method and does not include the so-called medical practice. In the cosmetic or non-therapeutic method according to the disclosure of the present invention, the composition for external use on the skin is applied to areas where desired effects are wished for, such as the face, neck, hands, arms, and legs. Application can be in any manner, including application by hand, applicator, and the like. The number, frequency, amount, and such of application are appropriately set according to the desired effect and the like.

### Preparations

1-(2-hydroxyethyl)-2-imidazolidinone or its derivatives and pyridinecarboxamide can be synthesized by known methods or they can be easily purchased commercially.

Also, 1-(2-hydroxyethyl)-2-imidazolidinone or its derivatives and pyridinecarboxamide can be converted into inorganic salts or organic salts by known methods. Salts used in the embodiments of the disclosure of the present invention are not particularly limited, and for example, inorganic salts include hydrochloride, sulfate, phosphate, hydrobromide, sodium salt, potassium salt, magnesium salt, calcium salts, ammonium salts and the like. Organic salts include acetate, lactate, maleate, fumarate, tartrate, citrate, methanesulfonate, p-toluenesulfonate, triethanolamine salt, diethanolamine salt, amino acid salt, and the like.

1-(2-hydroxyethyl)-2-imidazolidinone and its derivatives with a heparanase activity inhibiting effect and / or an MMP inhibitory effect, especially those with both heparanase activity inhibiting effect and MMP inhibiting effect (especially MMP-9 inhibiting effect), can be suitably used in the agents of the disclosure of the present invention. The composition according to the disclosure of the present invention may comprise only one type of 1-(2-hydroxyethyl)-2-imidazolidinone or a derivative thereof, and two or more types of the above compounds or salts thereof may be used in any optional combination or proportion.

The content of 1-(2-hydroxyethyl)-2-imidazolidinone or a derivative thereof and pyridinecarboxamide or a salt thereof in the compositions of the disclosure of the present invention is not particularly limited as long as the amount is sufficient to effectively produce the desired effect and should be selected according to the application of the agent. In general, however, the ratio of 1-(2-hydroxyethyl)-2-imidazolidinone or its derivative and pyridinecarboxamide or its salt to the total amount of the agent is usually 0.0001 % or more by mass, especially 0.0001 % or more by mass, and usually less than 1 % by mass, especially less than 0.2% by mass. When two or more 1-(2-hydroxyethyl)-2-imidazolidinone or its derivatives and pyridinecarboxamide or its salt are used, the total amount of them should meet the above range.

The compositions according to the disclosure of the present invention may also comprise any other optional ingredient, in addition to 1-(2-hydroxyethyl)-2-imidazolidinone or a derivative thereof and pyridinecarboxamide or a salt thereof, provided that the actions of 1-(2-hydroxyethyl)-2-imidazolidinone or a derivative thereof and pyridinecarboxamide or a salt thereof are not substantially impaired. The additional components include other compounds (CGS27023A, BIPBIPU, or such) having an inhibitory effect of a heparanase activity or any other activity (MMP inhibitory activity, especially MMP-9 inhibitory activity, or such), and pharmaceutically acceptable carriers and / or adjuvants. Examples of the additional ingredients include herbal medicines that act as heparanase inhibitors such as valerian extract, lily extract, chomeisou extract, soapberry extract, chimp extract (see Japanese Patent Application No. 2014-165586), mangosteen extract, turmeric extract, (tormentilla extract), and such (see Japanese Patent Application No. 2018-545781), but are not limited to thereto. Such additional components may be used alone with one single type, or two or more types may be used in any combination and proportion. Accordingly, some embodiments of the present invention relate to topical skin compositions comprising as active ingredients, pyridinecarboxamide and a compound having both heparanase inhibitory activity and MMP (especially MMP-9) inhibitory activity and / or a combination of a heparanase inhibiting agent and an MMP (especially MMP-9) inhibiting agent.

The compositions according to the disclosure of the present invention can be produced according to a conventional method, and they can also be prepared with only 1-(2-hydroxyethyl)-2-imidazolidinone or its derivatives and pyridinecarboxamide, or in addition with one or more of its salts, as ingredients that constitute a topical skin composition. Ingredients normally used in cosmetics, pharmaceuticals, and other topical skin care products, such as oils, surfactants, powders, colorants, water, alcohols, thickening agents, chelating agents, silicones, antioxidants, UV absorbers, moisturizing agents, fragrances, various medicinal ingredients, preservatives, pH adjusting agents, neutralizing agents, and the like are blended as needed.

The dosage form of the composition according to the disclosure of the present invention is not limited, and it may be appropriately selected according to the use thereof. Examples of administration routes include topical administration (external application to the skin). Formulations for topical administration (material for external application to the skin) include solution systems, solubilized systems, emulsified systems, powder dispersion systems, water-oil two-phase systems, water-oil-powder three-phase systems, or such, in patch form, ointment, cream, emulsion, lotion, gel, aerosol, and other forms.

Further, for the compositions according to the disclosure of the present invention, one or more optional components such as carriers and / or adjuvants may be blended in addition to the 1-(2-hydroxyethyl)-2-imidazolidinone or a derivative thereof and pyridinecarboxamide or a salt thereof according to the disclosure of the present invention, as long as the action of 1-(2-hydroxyethyl)-2-imidazolidinone or a derivative thereof and pyridinecarboxamide or a salt thereof is not substantially impaired. The additional ingredients are not particularly limited, and may be appropriately selected according to the use, dosage form, mode of administration of the pharmaceutical composition, and examples include pharmaceutically acceptable carriers and / or adjuvants. The adjuvants include, for example, diluents, binding agents, disintegrants, thickening agents, dispersants, reabsorption enhancers, flavoring agents, buffers, surfactants, solubilizers, preservatives, emulsifiers, isotonic agents, stabilizers, pH adjusters, and the like.

As specific examples, the compositions according to the disclosure of the present invention can comprise ingredients normally used in topical agents as needed, for example, whitening agents, moisturizing agents, antioxidants, oil-based ingredients, UV absorbers, surfactants, thickening agents, alcohols, powder ingredients, colorants, aqueous ingredients, water, various skin nutrition agents or such. In addition, metal ion sequestering agents such as disodium edetate, trisodium edetate, sodium citrate, sodium polyphosphate, sodium metaphosphate, and gluconic acid; preservatives such as methylparaben, ethylparaben, butylparaben and such; caffeine, tannin, verapamil, tranexamic acid and derivatives thereof, licorice extract, glabridine, hot water extract of the quince fruit, various herbal medicines, pharmaceutical agents such as tocopherol acetate, glycyrrhizic acid and its derivatives or salts, whitening agents such as vitamin C, magnesium ascorbate phosphate, ascorbic acid glucoside, arbutin, and kojic acid; saccharides such as glucose, fructose, mannose, sucrose, trehalose, and such; vitamin A derivatives such as retinoic acid, retinol, retinol acetate, retinol palmitate, and such can also be blended appropriately as needed.

The above ingredients are illustrative and not limiting. Also, these components can be blended in an appropriate combination according to the formulation depending on the desired form.

The dosage form of the topical skin preparations according to the disclosure of the present invention is not particularly limited, and as described above it can be, for example, a solution system, solubilized system, emulsion system, powder dispersion system, water-oil two-phase system, water-oil-powder three-phase system, ointment, gel, aerosol, or any other formulation. The form of use is also not limited particularly, and can take any optional form such as lotion, milky lotion, cream, essence, jelly, gel, ointment, pack, mask, foundation, or such.

By applying a composition according to the disclosure of the present invention to the skin, it can be used as a cosmetic method for improving the skin condition. The usage and dosage of the topical skin preparation according to the disclosure of the present invention in such cosmetic methods are not particularly limited, and they are appropriately determined depending on the dosage form and skin condition to be treated. Typically, a suitable amount, for example, 0.1 ml to 1 ml per square cm², can be rubbed directly into the skin, or the appropriate amount can be soaked into a gauze and applied to the skin several times per day, for example, one to five times per day.

The above description of the present invention with specific examples is only illustrative, and the invention can be implemented with any modification within the scope not departing from the claims. The various features and embodiments of the present invention referred to in various places above may be applied to the description of other parts of the present invention with the necessary changes as appropriate. Thus, features identified in one embodiment may be combined with features identified in the other embodiments as appropriate. All references cited herein, including patents, patent applications, articles, textbooks, and sequence accession numbers, and references cited therein are incorporated herein by reference in their entirety. To the extent that one or more of the incorporated references and similar materials differ from or contradict this application in any respect, including but not limited to defined terms, usage of terms, techniques described, and such, the description in the present application takes precedence.

Hereinafter, the present invention will be described more specifically with reference to examples, but the present invention is not limited to the examples below.

### Examples

### 1. Materials and method

### Culture of al three-dimensional epidermal skin model

The three-dimensional epidermal skin model (EPI-200-3S) purchased from MatTek was treated with nicotinamide (final concentration 7.5 × 10⁻³ M) and MMP-9 inhibitor CGS27023A (Reference 1) (final concentration 10⁻⁵ M), and it was cultured in a special medium (EPI-100 ASY) supplemented with the heparanase inhibitor BIPBIPU (Reference 2) (final concentration 10⁻⁵ M). As a control, the cells were cultured in a medium to which an equal volume of the DMSO solvent was added without these drugs. The medium was exchanged once every two days, and the pieces of tissue were collected on the fourth day.

### Culture of a three-dimensional skin model containing dermis

A dermis-containing three-dimensional skin model (EFT-400) purchased from MatTek was treated with nicotinamide (final concentration 7.5 × 10⁻³ M) and CGS27023A (final concentration 10⁻⁵ M), and it was cultured in a special medium (EPI-100 ASY) supplemented with BIPBIPU (final concentration 10⁻⁵ M). As a control, the cells were cultured in a medium to which an equal volume of the DMSO solvent was added without these drugs. The medium was exchanged once every two days, and the pieces of tissue were collected on the fourth day.

### Culture of fresh human skin

Fresh abdominal skin samples from subjects (20's to 30's) who gave informed consents were purchased from KAC Co., Ltd. They were cultured in a mixture medium of equal volumes of 10% FBS-DMEM (Thermo Fisher Science, 11885084) and Humedia-KG2 (KURABO, KK-2150S), supplemented with nicotinamide (7.5 × 10⁻³ M or 1.5 × 10⁻² M final concentration) and 1-(2-hydroxyethyl)-2-imidazolidinone (Reference 3) (S173, final concentration 0.01%), a proprietary ingredient that inhibits both MMP-9 and heparanase. As a control, the cells were cultured in a medium to which an equal volume of the DMSO solvent was added without these drugs. The medium was changed every day, and pieces of the tissue were collected on the second day.

### Paraffin block, sectioning

The recovered skin model and fresh human skin were dehydrated and fixed using cold acetone according to the AMeX method, then substituted with acetone, methyl benzoate and xylene in that order, and embedded in paraffin. Sections were prepared with a thickness of 3 pm, and sections for tissue staining were prepared.

### Various types of immunostaining

Paraffin sections with a thickness of 3 pm were deparaffinized with xylene and then hydrated with EtOH. Fluorescent immunostaining was performed using an Ki-67(SP6) antibody (Thermo Fisher Scientific, RM-9106-S, rabbit mouse monoclonal antibody) and an MCSP (melanoma-associated chondroitin sulphate proteoglycan) antibody (Millopore, MAB2029, 9.2.27, mouse monoclonal antibody).

For fluorescent immunostaining of various types of collagens, an antibody against Type I collagen (ROCKLAND, 600-401-103-0.5, rabbit polyclonal antibody), an antibody against Type II collagen (ROCKLAND, 600-401-105S, rabbit polyclonal antibody), an antibody against Type IV collagen (Progen, 10760, rabbit polyclonal antibody), and an antibody against Type V collagen (ORIGENE, AM10159PU-N, mouse monoclonal antibody) were used.

### Gene expression of MCSP

The collected fresh human skin was heat-treated on a hot plate at 60°C for 1 minute and then cooled on ice to separate the epidermis and dermis. The epidermis was placed in a 1 mL Trizol solution containing zirconia balls and was shaken for 3 minutes with a tissue pulverizer to crush the epidermis. RNA was extracted using chloroform and isopropanol, and the RNA was purified using the RNeasy mini kit (QIAGEN). After the RNA concentration was measured using NanoDrop, cDNA was synthesized using SuperScript VILO (Invitrogen). After that, the synthesized cDNA was used for quantitative PCR analysis which was performed using platinum SYBR green (Invitrogen). Table 1 shows the primers for the genes used.

**[Table 1]**

| Primer name | Sequence | SEQ ID NO |
|---|---|---|
| MCSP forward | CACGGCTCTGACCGACATAG | 1 |
| MCSP reverse | CCCAGCCCTCTACGACAGT | 2 |
| b2m forward | GTGGGATCGAGACATGTAAGCA | 3 |
| b2m reverse | CAATCCAAATGCGGCATCT | 4 |

Collagen gene expression analysis was performed in the same manner as described above using the separated dermis. Table 2 shows the primers used.

**[Table 2]**

| Primer name | Sequence | SEQ ID NO |
|---|---|---|
| Collagen 5A1 forward | GTGGCACAGAATTGCTCTCA | 5 |
| Collagen SA1 reverse | TCACCCTCAAACACCTCCTC | 6 |
| Pro collagen 1A1 forward | CTCGAGGTGGACACCACCCT | 7 |
| Pro collagen 1A1 reverse | CAGCTGGATGGCCACATCGC | 8 |
| Collagen 3A1 forward | TCCGGGTGAGAAAGGTGA | 9 |
| Collagen 3A1 reverse | GCAGGTCCAGAACCTCCAG | 10 |
| Collagen 4A1 forward | TGCGCAAGTTCAGCACAATG | 11 |
| Collagen 4A1 reverse | GGCACGGTGGGATCTGAATG | 12 |

### 2. Results and discussion

### Effect of nicotinamide and BIPBIPU + CGS on keratinocytes

Figure 1 shows the results of investigating the effect of nicotinamide (NAM) and the combination of MMP-9 inhibitor CGS27023A and heparanase inhibitor BIPBIPU on keratinocyte proliferation in a three-dimensional epidermal skin model. The results in Figure 1 show that treatment with nicotinamide (NAM) and CGS27023A + BIPBIPU increased the number of cells positive for the cell proliferation marker Ki-67.

In addition, the results of investigating the effect of nicotinamide (NAM) and the combination of MMP-9 inhibitor CGS27023A and heparanase inhibitor BIPBIPU on keratinocyte proliferation in a three-dimensional skin model containing dermis are illustrated in Figure 2. The results of Figure 2 show that treatment with nicotinamide (NAM) and CGS27023A + BIPBIPU increased the number of cells positive for the cell proliferation marker Ki-67.

### Effect of the combination of nicotinamide and S173 on keratinocytes

Figure 3 shows the results of H&E staining of the effect of nicotinamide (NAM) and 1-(2-hydroxyethyl)-2-imidazolidinone (S173) on fresh skin samples. The results in Figure 3 demonstrate that the combination of nicotinamide (NAM) and 1-(2-hydroxyethyl)-2-imidazolidinone (S173) (NAM + S173) preserved keratinocyte proliferation in basal layer cells. This is also supported by the increase in the number of cells positive for Ki-67, which is a cell proliferation marker, by the combination of nicotinamide (NAM) and 1-(2-hydroxyethyl)-2-imidazolidinone (S173) (NAM + S173), as shown in Figures 4 and 5. Thus, these results indicate that treatment with a combination of nicotinamide (NAM) and 1-(2-hydroxyethyl)-2-imidazolidinone (S173) (NAM + S173) promoted keratinocyte proliferation.

### Effect of the combination of nicotinamide and S173 on epidermal stem cells

Figures 6 and 7 show the results of examining the effect of nicotinamide (NAM) and 1-(2-hydroxyethyl)-2-imidazolidinone (S173) on epidermal stem cells. The results in Figures 6 and 7 show that treatment with nicotinamide (NAM) and 1-(2-hydroxyethyl)-2-imidazolidinone (S173) increased the number of cells positive for MCSP, which is an epidermal stem cell marker. Furthermore, Figure 8 shows the results of examining the expression level of the MCSP mRNA by qPCR analysis. The results in Figure 8 indicate that the combination of NAM and S173 (NAM + S173) synergistically increased the number of epidermal stem cells represented by the expression level of the MCSP mRNA. The above results demonstrate that the combined treatment with nicotinamide (NAM) and 1-(2-hydroxyethyl)-2-imidazolidinone (S173) promoted epidermal stem cell proliferation.

### Effect of the combination of nicotinamide and S173 on Type V collagen

Figures 9A, 9B and 9C show the results of examining the effect of nicotinamide (NAM) and 1-(2-hydroxyethyl)-2-imidazolidinone (S173) on the production of Type V collagen. Figure 9A shows the results of fluorescent immunostaining of Type V collagen. Figure 9B is a graph quantifying the intensity of staining divided by the area. Figure 9C is a graph showing relative expression levels of the collagen 5 A1 mRNA. The results in Figures 9A, 9B and 9C demonstrate that treatment with nicotinamide (NAM) and 1-(2-hydroxyethyl)-2-imidazolidinone (S173) increased the expression of Type V collagen. The results in Figures 9A, 9B and 9C show that the combination of NAM and S173 (NAM + S173) synergistically increased Type V collagen. These results indicate that treatment with a combination of nicotinamide (NAM) and 1-(2-hydroxyethyl)-2-imidazolidinone (S173) promoted the expression of collagen in the papillary dermis.

### Effect of the combination of nicotinamide and S173 on dermal collagen

Figures 10 to 12 show the results of investigating the effect of nicotinamide (NAM) and 1-(2-hydroxyethyl)-2-imidazolidinone (S173) on the production of dermal collagen. Figure 10A shows the results of fluorescent immunostaining of Type I collagen. Figure 10B is a graph quantifying the intensity of staining divided by the area. Figure 10C is a graph showing relative expression levels of the procollagen 1A1 mRNA. Figure 11A shows the results of fluorescent immunostaining of Type III collagen. Figure 11B is a graph quantifying the intensity of staining divided by the area. Figure 11C is a graph showing relative expression levels of the collagen 3 A1 mRNA. Figure 12A shows the results of fluorescent immunostaining of Type IV collagen. Figure 12B is a graph quantifying the intensity of staining divided by the area. Figure 12C is a graph showing the relative expression levels of the collagen 4A1 mRNA. The results in Figures 10 to 12 show that treatment with nicotinamide (NAM) and 1-(2-hydroxyethyl)-2-imidazolidinone (S173) resulted in increases of Type I collagen, Type II collagen, and Type IV collagen. The results in Figures 10 to 12 show that the combination of NAM and S173 (NAM + S173) synergistically increased the expression of dermal collagen. These results indicate that treatment with a combination of nicotinamide (NAM) and 1-(2-hydroxyethyl)-2-imidazolidinone (S173) promoted dermal collagen expression.

### References

1) Pan W, Miao HQ, Xu YJ, Navarro EC, Tonra JR, Corcoran E, et al. 1-[4-(1H-Benzoimidazol-2-yl)-phenyl]-3-[4-(1H-benzoimidazol -2-yl)-phenyl]-urea derivatives as small molecule heparanase inhibitors. Bioorganic & medicinal chemistry letters. 2006;16(2):409-12.
2) MacPherson LJ, Bayburt EK, Capparelli MP, Carroll BJ, Goldstein R, Justice MR, et al. Discovery of CGS 27023A, a non- peptidic , potent, and orally active stromelysin inhibitor that blocks cartilage degradation in rabbits. Journal of medicinal 1997;40(16):2525-32.
3) Iriyama S, Yamanishi H, Kunizawa N, Hirao T, Amano S. 1-(2-hydroxyethyl)-2-imidazolidinone, a heparanase and matrix metalloproteinase inhibitor, improves epidermal basement membrane structure and epidermal barrier function. Experimental dermatology. 2019 ;28(3):247-53.
4) Iriyama S, Yasuda M, Nishikawa S, Takai E, Hosoi J, Amano S. Decrease of laminin-511 in the basement membrane due to photoaging reduces epidermal stem/progenitor cells. Scientific reports. 2020;10(1):12592.

### Industrial Applicability

Since the compositions according to the disclosure of the present invention promote the proliferation of epidermal stem cells and facilitate the increase of keratinocytes, they can be suitably used, for example, as a cosmetic composition such as cosmetics effective for improving skin conditions.

## Claims

1. A topical skin composition comprising 1-(2-hydroxyethyl)-2-imidazolidinone or a derivative thereof and pyridinecarboxamide as active ingredients.

2. The composition according to claim 1, which is a cosmetic composition.

3. The composition according to claim 1 or 2, which is for use in promoting the proliferation of epidermal stem cells.

4. The composition according to claim 3, wherein the epidermal stem cells are MCSP-positive cells.

5. The composition according to any one of claims 1 to 4, which promotes keratinocyte proliferation.

6. The composition according to any one of claims 1 to 5, wherein the 1-(2-hydroxyethyl)-2-imidazolidinone or a derivative thereof is a cyclic carboxamide derivative represented by the general formula (I) below: 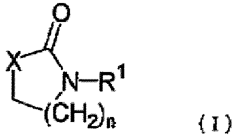 wherein, n is an integer of 1 to 3, R¹ is a hydrogen atom or a hydrocarbon group having 1 to 6 carbon atoms which may be substituted with a hydroxyl group, and X is -CH₂- or a group represented by -N(R²)-, and R² refers to a hydrogen atom or a hydrocarbon group having 1 to 6 carbon atoms which may be substituted with a hydroxyl group.

7. The composition according to any one of claims 1 to 6, wherein the pyridinecarboxamide is selected from the group consisting of 2-pyridinecarboxamide, 3-pyridinecarboxamide, 4-pyridinecarboxamide, and a mixture thereof.

8. A cosmetic method, comprising applying to the skin a composition comprising 1-(2-hydroxyethyl)-2-imidazolidinone or a derivative thereof and pyridinecarboxamide.

9. The composition according to any one of claims 1 to 7, which is for use in promoting the expression of collagen in the papillary dermis.

10. The composition according to any one of claims 1 to 7, which is for use in promoting the expression of collagen in the dermis.
